(19) Europäisches Patentamt
European Patent Office
Office européen des brevets

(11) EP 4 363 405 B1

(12) EUROPEAN PATENT SPECIFICATION

(45) Date of publication and mention of the grant of the patent:
**21.05.2025 Bulletin 2025/21**

(21) Application number: **22738634.9**

(22) Date of filing: **28.06.2022**

(51) International Patent Classification (IPC):
***C07D 233/86*** (2006.01) ***C07C 237/00*** (2006.01)
***C07C 231/12*** (2006.01) ***C07C 237/30*** (2006.01)

(52) Cooperative Patent Classification (CPC):
(C-Sets available)
**C07D 233/86; C07C 231/12; C07C 237/30** (Cont.)

(86) International application number:
**PCT/EP2022/067799**

(87) International publication number:
**WO 2023/275091 (05.01.2023 Gazette 2023/01)**

(54) **PROCESSES FOR THE PREPARATION OF NON-STEROIDAL ANTIANDROGENS**

VERFAHREN ZUR HERSTELLUNG NICHTSTEROIDALER ANTIANDROGENE

PROCÉDÉS DE LA PRÉPARATION D’ANTIANDROGÈNES NON STÉROÏDIENS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.06.2021 EP 21382571**

(43) Date of publication of application:
**08.05.2024 Bulletin 2024/19**

(73) Proprietor: **Química Sintética, S.A.**
**28805 Madrid (ES)**

(72) Inventors:
- **BARRECA, Giuseppe**
**23896 Sirtori (IT)**
- **LIMING, Huang**
**Hangzhou City, Zhejiang 311500 (CN)**

(74) Representative: **Ponti & Partners, S.L.P**
**Edifici PRISMA**
**Av. Diagonal núm. 611-613, Planta 2**
**08028 Barcelona (ES)**

(56) References cited:
**WO-A1-2006/124118**
**WO-A1-2011/106570**
**WO-A1-2015/121768**
**WO-A1-2015/154730**
**CN-A- 108 069 869**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 231/12, C07C 237/30**

## Description

### Field of the invention

The present invention relates to the preparation of non-steroidal antiandrogen drugs, such as 4-[3-[4-cyano-3-(trifluoromethyl)phenyl]-5,5-dimethyl-4-oxo-2-thioxo-1-imidazolidinyl]-2-fluoro-N-methyl-benzamide and intermediates thereof.

### Background

4-[3-[4-Cyano-3-(trifluoromethyl)phenyl]-5,5-dimethyl-4-oxo-2-thioxo-1-imidazolidinyl]-2-fluoro-N-methyl-benzamide (enzalutamide, marketed under the brand name Xtandi®), is a nonsteroidal antiandrogen (NSAA) medication indicated for use in the treatment of metastatic castration-resistant prostate cancer (mCRPC) and non-metastatic castration-resistant prostate cancer. It is taken by oral administration.

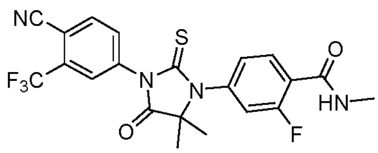

Enzalutamide was first described in 2006, and was introduced for the treatment of prostate cancer in 2012. It was the first NSAA of second generation to enter the market. The medication is widely available throughout the world.

Enzalutamide was approved by the U.S. Food and Drug Administration (FDA) on August 31, 2012, then approved by European Medicine Agency (EMA) on June 21, 2013, and approved by Pharmaceuticals and Medical Devices Agency of Japan (PMDA) on March 24, 2014.

A first route of synthesis for enzalutamide was disclosed in WO 2006/124118. Due to the scarce selectivity of the last coupling step, this synthetic approach was deemed unsuitable for industrial production purposes.

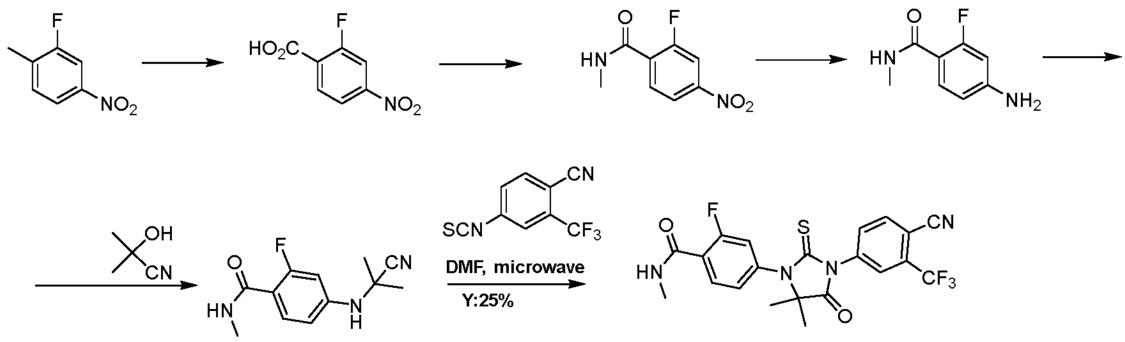


Numerous synthetic strategies have been proposed over years for the preparation of enzalutamide, mostly entailing a convergent approach. Of particular relevance is that described in WO 2011/106570, wherein the thiohydantoin ring can be prepared through the coupling of two key fragments, referred to in the following as fragment A and fragment B.

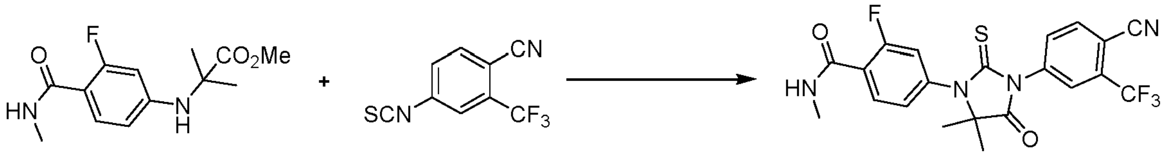

**Fragment A** **Fragment B**

It was experimentally found that said procedure suffers of significant disadvantages concerning the use of a large excess of fragment B with the respect to fragment A, to obtain the product in acceptable industrial yield which impacts on the overall cost of the process and purity of the final product.

WO 2015/121768 describes a process comprising a coupling reaction between fragment B and fragment A', wherein in said fragment X is chosen in the group consisting of methyl, ethyl, isopropyl, t-butyl, phenyl, or benzyl. In the example 3 of WO 2015/121768 only the coupling of isothiocyanate with fragment A', wherein X is an ethyl group is reported. The reaction in said example is carried out using 2 equivalents of fragment B respect to fragment A' with 60.7% of total yield.

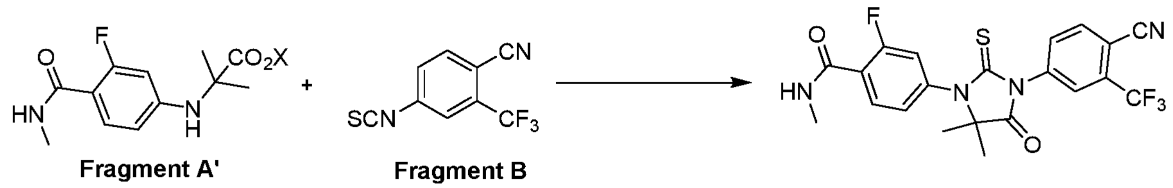

**Fragment A'** **Fragment B**

WO 2015/154730 discloses a process wherein the coupling is between fragment A-acid and fragment B, as shown below.

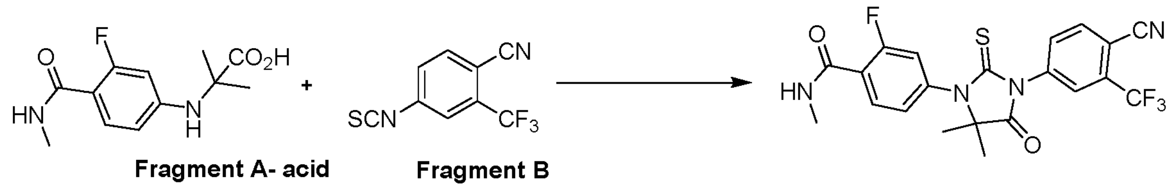

**Fragment A- acid** **Fragment B**

Said reaction is carried out in presence of a large excess of phenol (up to 6 equivalents with respect to fragment A-acid), in order to minimize the formation of impurity indicated as "oxo-enzalutamide", having the structure reported here below, and resulting from the oxidation of enzalutamide.

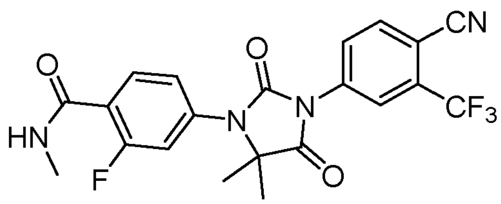

oxo-enzalutamide

A significant drawback of this method is linked to the use of large quantities of phenol, which is corrosive, highly irritating and toxic to humans, hence this method entails safety issues, which make it unsuitable for large scale production.

An object of the present invention is, therefore, to provide a synthetic approach to the preparation of enzalutamide, and related structures, that is cost-effective, with improved safety and feasible on large scale, minimizing or avoiding the formation of impurities deriving from side-reactions.

**Summary of the invention**

These objectives, together with other objectives which will be made evident hereafter, are achieved by the present invention that, in an aspect thereof, relates to a process for the preparation of a compound having the structure of formula (I)

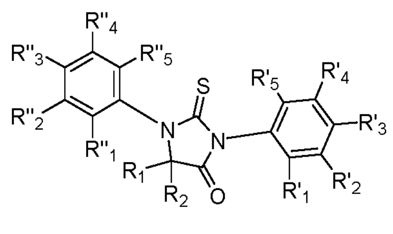

**(I)**

wherein:

- $R_1$ and $R_2$, each independently from the other, can be H, $C_1$-$C_8$-alkyl or $C_6$-$C_{10}$-aryl, optionally substituted with one or more halide, cyano, hydroxy or amino group(s);
- one or more of $R'_1$ - $R'_5$ and of $R''_1$- $R''_5$, each independently from the others, is selected from the group consisting of $R_1$, cyano, $-COOR_1$, $-CONR_1R_2$, $-OCOR_1$, - $OCNR_1R_2$, wherein $R_1$ and $R_2$ are as defined above,
- the process comprising the step of coupling the compound of formula (II) and the compound of formula (III) to obtain the compound of formula (I)

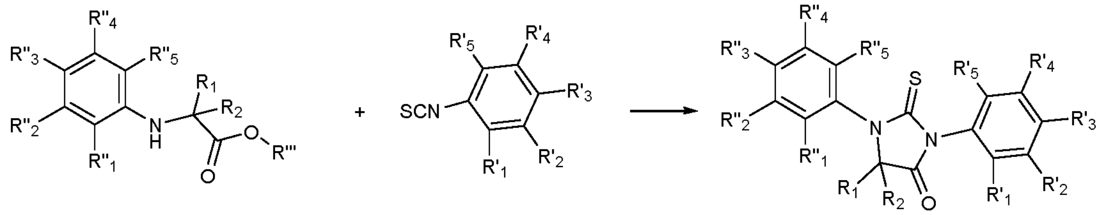

(II) (III) (I)

- wherein R‴ is a phenyl bearing one or more substituent(s) independently selected from halide and $-NO_2$.

The meaning of R'1 - R'5 and of R"1 - R"5 defined above are applicable indistinctly in formulae (I), (II) and (III), or a derivate thereof.

In another aspect, the present invention relates to the compound of general formula (IIa)

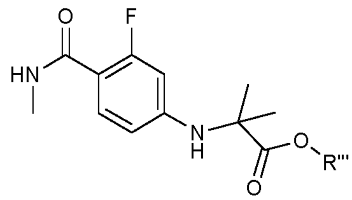

(IIa)

wherein R‴ is a phenyl bearing one or more substituent(s) independently selected from halide and $-NO_2$.

Preferably, R‴ is 4-nitrophenyl, named herein compound (IIb), or 4-chlorophenyl, named herein compound (IIc):

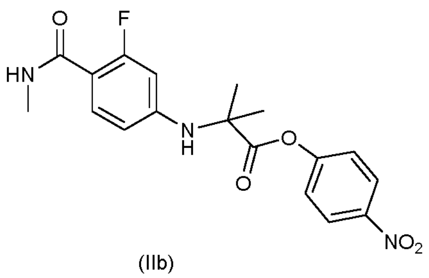

(IIb)

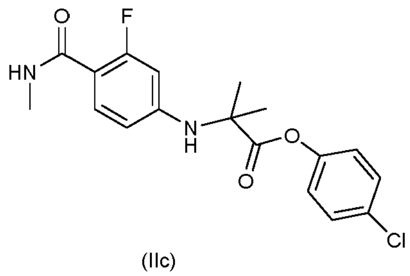

(IIc)

In another aspect, the present invention relates to a process for the preparation of the compound of general formula (IIa) as described above, comprising the step of esterification of the acid of formula (IV) with a phenol derivative of formula R'''OH, wherein R‴ is as defined above, preferably wherein R‴ is 4-nitrophenyl.

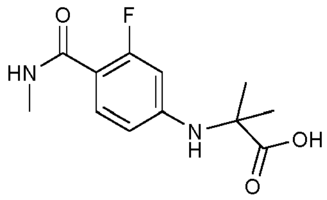

(IV)

In an aspect, the present invention relates to the use of a compound of general formula (IIa) or compound of formula (IIb) or compound of formula (IIc) for the preparation of enzalutamide.

**Detailed description of the invention**

All terms used in this application, unless otherwise specified, are to be understood in their ordinary meaning as known in the technical field.

The term *"about"* includes the range of experimental errors, which can normally occur performing a measurement, e.g. ± 5% or ±2% or ±1 %.

The term *"mass"* defines the combination of substrates, reagents, solvents, and products on which a physical or chemical transformation is carried out.

Unless otherwise indicated, in the context of the present invention the percentage and amount of a certain component in a composition are to be referred to the weight of said component with respect to the total weight of the composition.

Unless otherwise indicated, in the context of the present invention the indication that a composition *"comprises"* other one or more components/elements means that the indicated components/elements must be present and also other components may be present, but are not necessarily present, in the composition, in addition to the ones specifically recited. In other words, the indication that a composition *"comprises"* one or more components does not exclude that the composition *consists* of, or consists essentially of, the recited component(s). Similarly, the indication that a process "comprises" one or more steps does not exclude that the process comprises steps, such as synthetic steps or purification steps, in addition to the one or more steps explicitly recited.

As used herein, the indication that a compound or composition A is *"entirely free"* of other substances (or *"consists of')* or the term "nd" means that, within the detection range of the instrument or method being used, no substances other than those specifically indicated can be detected in A.

Unless otherwise indicated, in the context of the present invention a range of values indicated for a certain parameter, for example the weight of a component in a mixture, includes the upper and the lower limits of the range, e.g. if the content in weight, or in volume, of a component A in a mixture is indicated as *"X to Y",* the content of A can be X, Y or any of the intermediate values.

In one aspect, the present invention relates to a process for the preparation of a compound having the structure of formula (I)

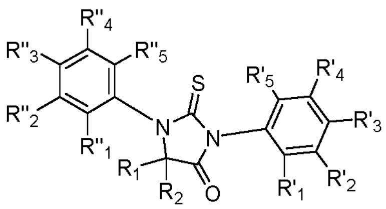

**(I)**

wherein:

- $R_1$ and $R_2$, each independently from the other, can be H, $C_1$-$C_8$ alkyl or aryl, optionally substituted with one or more halide, cyano, hydroxy or amino group(s);
- one or more of $R'_1$ - $R'_5$ and of $R''_1$ - $R''_5$, each independently from the others, is selected from the group consisting of $R_1$, cyano, halide, $-COOR_1$, $-CONR_1R_2$, - $OCOR_1$, $-OCNR_1R_2$, wherein $R_1$ and $R_2$ are as defined above,
- the process comprising the step of coupling the compound of formula (II) and the compound of formula (III) to obtain

the compound of formula (I)

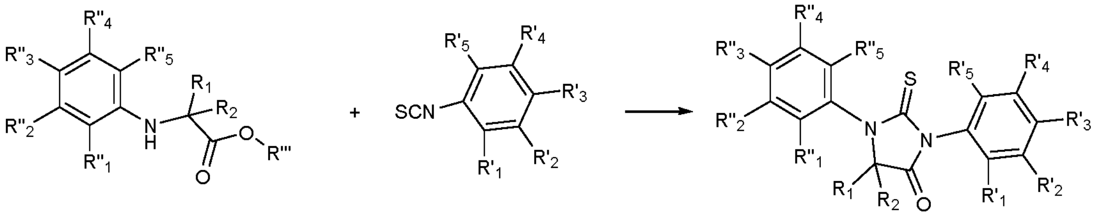

(II) (III) (I)

- wherein R''' is a phenyl bearing one or more substituent(s) independently selected from halide and $-NO_2$.

Preferably, in the process according to the present invention, at least one of R"1-R"5 is an halide or -CONHR1, wherein the halide is a fluorine group and R1 is as defined above .

Preferably, in the process according to the present invention, at least one of R'1-R'5 is a trifluoromethyl or a cyano group.

The above process is suitable to be used for the preparation of 4-[3-[4-cyano-3-(trifluoromethyl)phenyl]-5,5-dimethyl-4-oxo-2-thioxo-1-imidazolidinyl]-2-fluoro-N-methyl-benzamide (enzalutamide) and of several analogues thereof.

Upon analysing the known approaches in the art, in particular those of WO 2011/106570 and WO 2015/121768, it was found that the coupling of fragments A or A' and B requires at least 2 equivalents of the latter (namely the one bearing the isothiocyanate moiety) to obtain the final product in good and acceptable yields necessary to develop the process on an industrial scale. Without intending to be bound by theory, this is likely due to the tendency of the methanol, produced during the course of the reaction, to react with fragment B itself. This results in the formation of the corresponding methyl thiocarbamate, an impurity which must be removed from the reaction product, and ultimately subtracts fragment B from the reaction mixture. Furthermore, it was found that the presence of a large amount of fragment B in solution leads to an increase of impurities related to the side reactions of said fragment. The impurities called Thiourea, Urea and Trimer Guanidine, reported herein below are generated by side reaction of fragment B. In particular, Thiourea is generated from the coupling of fragment B and the corresponding aniline obtained from the hydrolysis of isothiocyanate moiety, Urea is the oxidation product of Thiourea impurity and Trimer Guanidine is the by-products obtained from the self-condensation of fragment B.

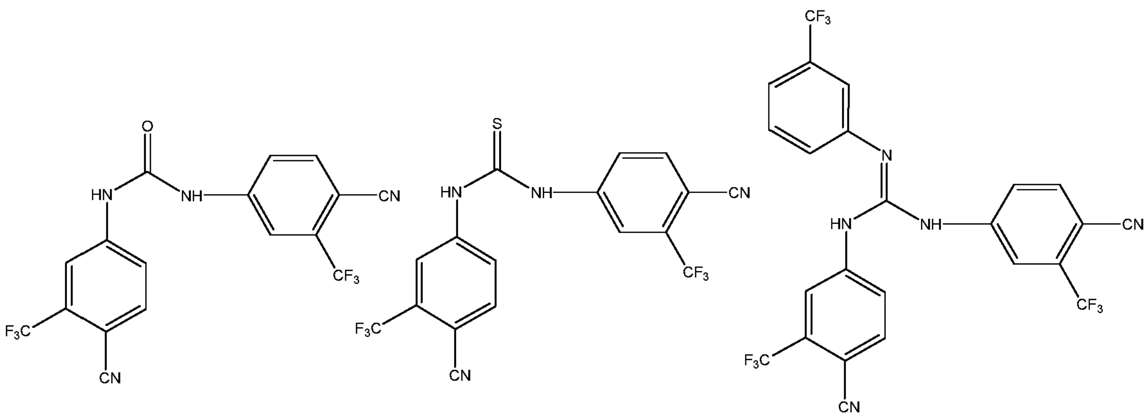

Urea Thiourea Trimer Guanidine

In addition being fragment B the reagent which most significantly impacts on the overall cost of the process, also for the use for its preparation of thiophosgene (a highly toxic compound requiring specific equipment for its production and manipulation) or a derivative thereof, the overall cost and feasibility of the WO 2011/106570 and WO 2015/121768 procedures is found to be far from ideal.

The strategy involving the coupling of fragment B with fragment A-acid, following the approach of WO 2015/154730, was initially investigated. This approach was found to be scarcely selective, both on small and on a multigram scale, in that it leads to the formation, to a significant extent, of at least two impurities related to fragment A-acid and very difficult to eliminate by crystallization

Said impurities, referred to *"open-ring impurity"* (15-20% HPLC area with respect to Enzalutamide), having the

structure shown in the following figure, and *"oxo-enzalutamide"* (2-3% HPLC area with respect to Enzalutamide),

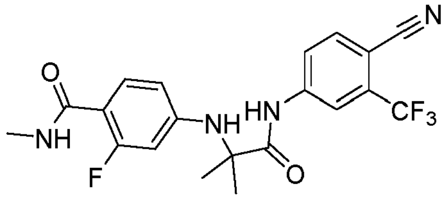

Open-ring impurity

Accordingly, a multi-stage purification process was developed to reduce their content below the acceptance limit, thus resulting in an overall yield (referred to the preparation and purification of enzalutamide) of less than 60% with respect to fragment A-acid.

Oxo-Enzalutamide turned out to be hardly purgeable by crystallization. Furthermore, any attempts to prevent its formation by modifying the reaction parameters failed. Its removal could be successfully achieved through crystallization from a methanol/water mixture (as described in Cryst. Growth. Des 2018, 18 (7), 3774-3780). It was, however, found that, while amounts of this impurity of about 2% can be effectively purged with a single crystallization procedure, the same crystallization conditions are not as effective in case of higher starting quantities, hence multiple crystallizations may be needed, with consequent loss of yield and diminished efficiency of the overall process.

It was further found that using fragment A-acid the consumption of the most expensive reagent fragment B has been significantly reduced with respect to the amounts required in WO 2011/106570 and WO 2015/121768 and that by adding a large amount of phenol (up to 6 equivalents with respect to fragment A-acid) the impurity oxo-enzalutamide became lower.

The possibility of replacing fragment A with an aromatic ester defined as compound (II) in the coupling reaction was then investigated, to understand if it is possible to reduce the equivalents of fragment B bearing the isothiocyanate moiety and if the presence of free phenol, generated after the cyclization reaction in a stoichiometric quantity, not in large amount as taught by WO 2015/154730, could prevent the formation of oxoenzalutamide impurity. Several aromatic esters were thus prepared using a series of phenols and aromatic compounds bearing an OH group, optionally substituted with electron-withdrawing or electron-donating groups, and tested in the coupling reaction with the fragment B.

Advantageously, in the process according to the present invention, R‴ is phenyl bearing one or more substituent(s) independently selected from an halide and $-NO_2$.

Preferably, in the process according to the present invention, the compound (II) has the following general formula (IIa):

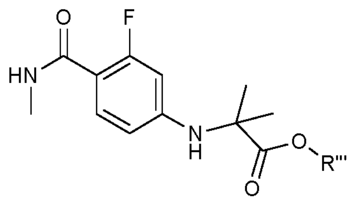

(IIa)

wherein R‴ is a phenyl bearing one or more substituent(s) independently selected from halide and $-NO_2$

The 4-nitrophenol and 4-chloro phenolester of fragment A are particularly preferred in view of the fact that the said ester are obtained with a good yield, in a crystalline easily purifiable form and lead to obtain the desired cyclisation product (compound I) with a high degree of purity and conversion .

Preferably, the present invention relates to the process as described above, wherein compound (II) has the following formula (IIb):

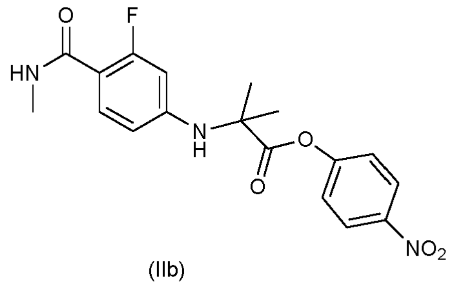

(IIb)

in view of the fact that the corresponding nitro-phenol is widely commercially available and scarcely toxic to handle in order to perform the process in an industrial scale.

More preferably, the present invention relates to the process as described above, wherein compound (III) has the following formula (IIIa):

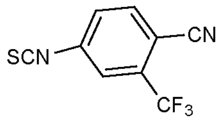

(IIIa)

Advantageously, the present invention relates to the process as described above wherein in the step of coupling the compound of formula (IIb) is coupled with the compound of formula (IIIa) and 4-[3-[4-cyano-3-(trifluoromethyl) phenyl]-5,5-dimethyl-4-oxo-2-thioxo-1-imidazolidinyl]-2-fluoro-N-methyl-benzamide is obtained and, optionally, purified via crystallization.

The advantages of the synthesis according to the present invention were clearly shown in a series of experiments carried out following the teaching of WO 2011/106570.

Said teaching was chosen instead of that written in WO 2015/121768, because, even if similar starting material have been involved in both processes, this process leads to obtain enzalutamide in higher yield.

Specifically, two sets of experiments, one relative to the synthesis of the present invention and one following the approach of WO 2011/106570, i.e. coupling using the methyl ester fragment A, were conducted, following the general coupling procedure disclosed in example 5 of WO 2011/106570 and varying the amount of fragment B, herein named compound (IIIa), with respect to fragment A with the aim of finding the minimum amount of compound (IIIa) necessary to convert completely fragment A into the final product. The purity profile, and the yield, calculated by titration in the reaction mixture, obtained with both synthetic approaches were evaluated.

For the procedure of WO 2011/106570 (coupling using the fragment A as methyl ester) it was confirmed that: Complete conversion of fragment A is achieved only when 2.5 equivalents of compound (IIIa) are used, with yield of about 95%.

- With 2 equivalents of compound (IIIa) (i.e. the amount reported in the example 5 of WO 2011/106570), the titrated yield is about 91% as reported in comparative example 6 of the experimental part.
- With lower amounts of compound (IIIa) the reaction does not proceed to complete conversion of fragment A
- The methyl thiocarbamate impurity is formed since the beginning of the reaction. Its formation might explain the significant consumption of compound (IIIa).

Adding a large excess of compound (IIIa), which would allow to convert completely fragment A into the final product, is the cause of the formation of a large amount of impurities related to compound (IIIa) degradation. The approach according to the present invention was carried out following the general procedure in example 5 of WO 2011/106570 and using, as non-limiting example, the 4-nitrophenol ester compound (IIb) as shown hereunder:

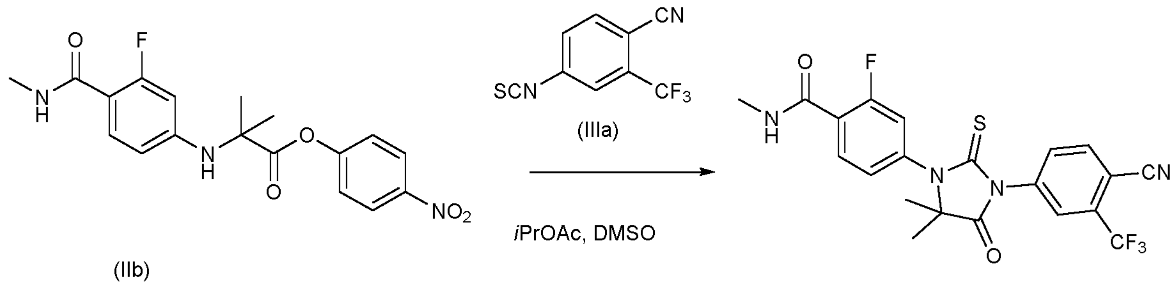


(IIb)

The following was observed:

- Complete conversion of compound (IIb) is achieved when from 1 to about 1.4 equivalents, preferably from 1.2 to 1.3 equivalents, of compound (IIIa) with respect to compound (IIb) are used, and the corresponding yield resulted to be from about 90 about 95%. In comparative example 7 of experimental part are reported the impurity profile and the titrated yield obtained using 1.2 and 1.4 equivalent of compound (IIIa).
- With a slightly higher amount of compound (IIIa), the reaction reaches the endpoint faster however an increase of the impurities related to compound (IIIa) is showed.

Thus, the synthetic route according to the present invention allows to produce enzalutamide with yield comparable to that of the process disclosed in WO 2011/106570 and using approximately half the amount of compound (IIIa), consequently, lowering the amount of by products and improving the cost-effectiveness of the process, as demonstrated by the comparison of High liquid chromatography (HPLC) data reported in example comparative 6 and 7.

Furthermore, by analysing the amount of oxo-enzalutamide in the final enzalutamide samples obtained in both stet of experiments, it is evident that when compound IIb is used as reactant the level of said impurity is lowered.

Preferably, the process of the present invention is further comprising the step of preparing the compound of formula (II) by esterification of the corresponding carboxylic acid with phenol derivative of formula R'''OH, wherein R''' is a phenyl bearing one or more substituent (s) independently selected from halide and -$NO_2$, optionally in the presence of one or more condensation agent(s).

Among the known methods to prepare phenol esters, those involving the use of a coupling agent were found to ensure optimal selectivity and improved purity of the reaction mixture. Among the common coupling agents (included, i.e., carbonyl diimidazole), the best results were achieved using N,N'-dicyclohexylcarbodiimide (DCC) and 1-ethyl-3-(3-dimethylaminopropyl)carbodiimide hydrochloride (EDAC). Due to some issues related to the work-up (DCC forms an insoluble and hardly removable urea), EDAC is preferred as the coupling agent to be used since the corresponding urea shows high solubility in water (thus allowing for an easy removal thereof through aqueous work-up).

The coupling of compound of formula (IV) and a phenol derivative of formula R'''OH, in the presence of EDAC proceeds even in the absence of catalysts but the use of catalytic amounts of strong organic bases, e.g. 4-dimethylaminopyridine (DMAP), result in a significant increase of the reaction rate, limiting, at the same time, the formation of the corresponding N-acylureas (known by-products of this kind of reactions) thus increasing the yield.

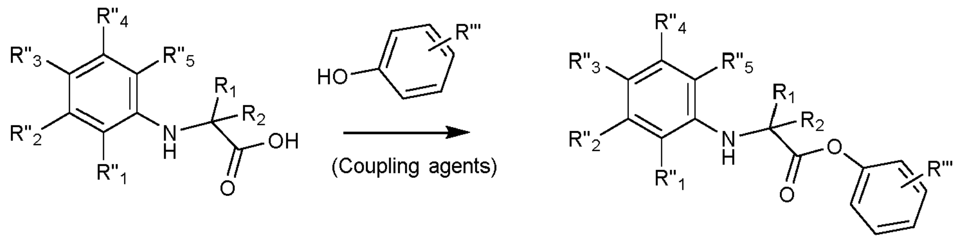


(IV) (II)

In another aspect, the present invention relates to a compound of general formula (IIa)

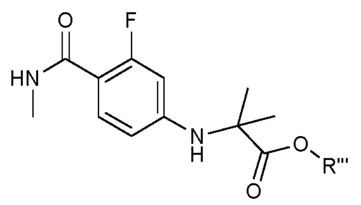

(IIa)

wherein R‴ is a phenyl bearing one or more substituent(s) independently selected from halide and $-NO_2$.

More preferably, R‴ is 4-nitrophenyl, 4-chlorophenyl pentafluorophenyl, 2,4-dichlorophenyl or 2,4-difluorophenyl. Still more preferably, R‴ is 4-nitrophenyl or 4-chloropheny. Advantageously in the compound of general formula (IIa), the R‴ group is 4-nitrophenyl.

In yet another aspect, the present invention relates to a process for the preparation of the compound of general formula (IIa) as described above, comprising the step of esterification of the acid of formula (IV) with a phenol derivative of formula R"'OH, wherein R‴ is as defined above, preferably wherein R‴ is 4-nitrophenyl.

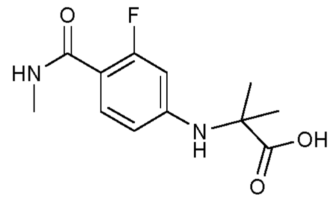

(IV)

In a further aspect, the present invention relates to the use of a compound of general formula (IIa) or compound of formula (IIb) or compound of formula (IIc) for the preparation of enzalutamide.

The following examples are provided to illustrate specific embodiments of the present invention, without intention to limit its scope.

**Example 1:** Preparation of 4-nitrophenyl 2-((3-fluoro-4-(methylcarbamoyl)phenyl)amino)-2-methyl propanoate, compound of formula (IIb).

In a 2-liter jacketed glass reactor equipped with mechanical stirrer, thermometer, reflux condenser and kept under nitrogen atmosphere are charged EDAC (1-ethyl-3-(3-dimethylaminopropyl)carbodiimide hydrochloride, 90 g), DMAP (N,N-dimethylaminopyridine, 3.4 g) and acetone (300 ml). The suspension is cooled under stirring to about -10°C.

In another 500-ml jacketed glass reactor equipped with mechanical stirrer, thermometer, reflux condenser and kept under nitrogen atmosphere are charged 2-((3-fluoro-4-(methylcarbamoyl)phenyl)amino)-2-methylpropanoic acid (100 g), 4-nitrophenol (60 g) and acetone (200 ml). The suspension is cooled under stirring to about 0°C and slowly transferred portion-wise, in about 15 minutes, into the reactor containing the suspension of EDAC, DMAP and acetone.

The reaction mixture is kept under stirring at -10/-5°C until reaction is complete.

When the reaction is over (HPLC), to the mixture maintained under stirring at 0-5°C, water (900 ml) is added dropwise, in not less than 30 minutes, while keeping internal temperature below 15°C. After about 50% of water addition, a white solid starts to precipitate.

The resulting suspension is kept under stirring at 0-5°C for at least 2 hours, filtered and reactor and the cake are washed with water.

Wet crude (IIb) (about 210 g) is used as it is for the following purification step.

Basing on the loss on drying the corresponding dry product corresponds to about 135 g (91% of the theoretical yield).

In a 2-liters jacketed glass reactor equipped with mechanical stirrer, thermometer, reflux condenser and kept under nitrogen atmosphere are charged 210 g of wet crude (IIb) (all the amount coming from the previous step), acetone (600 ml) and water (100 ml).

The suspension is heated under stirring to 50-55°C until almost all the solid is dissolved and a slight turbidity is present. Solution is filtered through a pad of celite and charcoal and combined with the previous filtered solution.

The clean solution of (IIb) is charged in the same reactor, and while keeping internal temperature at 50-55°C water (800 ml) is added dropwise, in not less the 30 minutes.

After about 50% of the addition precipitation of a white solid occurs. The suspension is cooled in about 2 hours to

0-5°C, kept under stirring in these conditions for not less than 2 hours and filtered. Reactor and the cake are washed with water.

The wet product (about 150 g) is dried under vacuum at 40-45°C until residual water (by KF analysis) is less than 0.2%. 125 g of (IIb, 85% yield) are obtained.

**Example 2:** Preparation of 4-[3-[4-cyano-3-(trifluoromethyl)phenyl]-5,5-dimethyl-4-oxo-2-thioxo-1-imidazolidinyl] -2-fluoro-N-methyl-benzamide (Enzalutamide).

In a 2-liter jacketed glass reactor, equipped with mechanical stirrer, thermometer, condenser and kept under nitrogen atmosphere are charged compound of formula (IIIa) (4-isothiocyanato-2-(trifluoromethyl)benzonitrile, 106 g, obtainable as disclosed in WO 2011/106570), compound of formula (IIb) (125 g), isopropyl acetate (250 ml) and DMSO (125 ml).

The suspension is heated under stirring to 73-75°C and kept in these conditions for at least 24 hours before sampling for analysis. A solution is obtained.

When the reaction is complete (e.g. via HPLC analysis), the reaction solution is cooled to about 45-50°C, methanol (25 ml) is added and the mixture is kept in these conditions for 1 hour. While keeping internal temperature at 40-45°C isopropyl acetate (520 ml) and water (250 ml) are added.

The mixture is kept under stirring at 40-45°C for at least 15 minutes, the agitation is stopped and the layers are left to separate. The aqueous layer is discarded and the organic layer is washed at 40-45°C with water (250 ml).

The washed organic solution is concentrated under vacuum until a thick suspension is obtained. Methanol (250 ml) is added to the residue and the mixture is distilled in the same conditions until a thick residue is obtained.

Methanol (850 ml) is added to the residue and the mixture is heated under stirring to reflux until a solution is achieved and water (170 ml) is slowly added by maintaining internal temperature above 55°C.

The mixture is cooled to 40-45°C and kept under stirring in these conditions until abundant crystallization occurs (about 30 minutes), then stirred at 0-5°C and filtered.

The solid thus obtained can be further crystallized from isopropanol and water to obtain 115 g of dry enzalutamide as white crystals in the crystalline form R1 (75% molar yield from compound of formula (IIb)). Purity 99.8 measured by HPLC analysis.

**Example 3:** Preparation of 4-chlorophenyl 2-((3-fluoro-4-(methylcarbamoyl)phenyl)amino)-2-methyl propanoate, compound of formula (IIc).

In a 5-liter jacketed glass reactor equipped with mechanical stirrer, thermometer, reflux condenser and kept under nitrogen atmosphere are charged EDAC (211 g), DMAP (12.5 g) and acetone (1200 ml)

The suspension is cooled under stirring to about -5°C.

In another 1L jacketed glass reactor equipped with mechanical stirrer, thermometer, reflux condenser and kept under nitrogen atmosphere are charged 2-((3-fluoro-4-(methylcarbamoyl)phenyl)amino)-2-methylpropanoic acid (200 g), 4-chlorophenol (111.2 g) and acetone (750 ml). The suspension is cooled under stirring to about 0°C and slowly transferred portion-wise, in about 15 minutes, into the reactor containing the suspension of EDAC, DMAP and acetone.

The reaction mixture is kept under stirring at -10/-5°C until reaction is complete.

When the reaction is over (HPLC), to the mixture maintained under stirring at 0-5°C, water (2000 ml) is added dropwise, in not less than 30 minutes, while keeping internal temperature below 15°C. After about 50% of water addition, a white solid starts to precipitate.

The resulting suspension is kept under stirring at 0-5°C for at least 2 hours, filtered and reactor and the cake are washed with water.

The wet crude (about 400 g) is used as it is for the following purification step.

In a 5-liters jacketed glass reactor equipped with mechanical stirrer, thermometer, reflux condenser and kept under nitrogen atmosphere are charged 400 g of wet crude (from the previous step), acetone (600 ml) and water (100 ml).

The suspension is heated under stirring to 50-55°C until almost all the solid is dissolved and a slight turbidity is present. The solution is filtered through a pad of celite and charcoal and combined with the previous filtered solution.

The so-obtained clean solution is charged in the same reactor, and, while keeping internal temperature at 50-55°C water (1200 ml) is added dropwise, in not less the 30 minutes.

After about 50% of the addition precipitation of a white solid occurs. The suspension is cooled in about 2 hours to 0-5°C, kept under stirring in these conditions for not less than 2 hours and filtered. Reactor and the cake are washed with water.

The wet product (about 280 g) is dried under vacuum at 40-45°C. 237 g of the tile product are obtained (83% yield).

**Example 4:** Preparation of 4-[3-[4-cyano-3-(trifluoromethyl)phenyl]-5,5-dimethyl-4-oxo-2-thioxo-1-imidazolidinyl] -2-fluoro-N-methyl-benzamide (Enzalutamide).

In a 5-liter jacketed glass reactor, equipped with mechanical stirrer, thermometer, condenser and kept under nitrogen atmosphere are charged compound of formula (IIIa) (4-isothiocyanato-2-(trifluoromethyl)benzonitrile,163 g, obtainable as disclosed in WO 2011/106570), 4-chlorophenyl 2-((3-fluoro-4-(methylcarbamoyl)phenyl)amino)-2-methyl

propanoate (200 g), isopropyl acetate (400 ml) and DMSO (200 ml).

The suspension is heated under stirring to 60-70°C and kept in these conditions for at least 20 hours before sampling for analysis. A solution is obtained.

When the reaction is complete (e.g. via HPLC analysis), the reaction solution is cooled to about 45-50°C, methanol (30 ml) is added and the mixture is kept in these conditions for 1 hour. While keeping internal temperature at 40-45°C isopropyl acetate (1000 ml) and a 5% sodium carbonate solution in water (600 ml) are added.

The mixture is kept under stirring at 20-25°C for at least 15 minutes, the agitation is stopped and the layers are left to separate. The aqueous layer is discarded and the washed organic solution is concentrated under vacuum until a thick suspension is obtained. Methanol (400 ml) is added to the residue and the mixture is distilled in the same conditions until a thick residue is obtained.

Methanol (1400 ml) is added to the residue and the mixture is heated under stirring to reflux until a solution is achieved and a seed of crystalline enzalutamide (crystalline form R2) is added at 38-40°C and kept under stirring in these conditions until abundant crystallization occurs (about 30 minutes), then stirred at 0-5°C and filtered.

The solid thus obtained can be further crystallized from isopropanol and water to obtain 201 g of dry enzalutamide as white crystals in the crystalline form R1 (79% molar yield from 4-chlorophenyl 2-((3-fluoro-4-(methylcarbamoyl)phenyl) amino)-2-methyl propanoate) **Purity 99.9** % measured by HPLC analysis

**Example 5:** The following intermediates of general formula (IIa) were prepared starting from the corresponding acid (compound IV) following the procedure reported in example 1 and 3 and used in the preparation of enzalutamide, with the overall yield for the preparation of enzalutamide (starting from 2-((3-fluoro-4-(methylcarbamoyl)phenyl)amino)-2-methylpropanoic acid) shown in the table below.

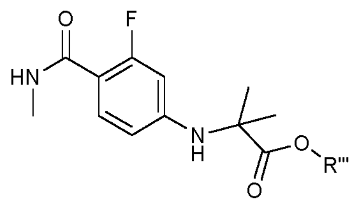

(IIa)

| R‴ is a phenyl bearing one substituent selected from the group consisting of: | Enzalutamide synthesis Overall yield% |
|---|---|
| Penta-F | 88.2 |
| 2,4-di-Cl | 71.2 |
| 2,4-Di-F | 77.9 |
| 2-Cl | 73.7 |
| 4-Br | 73.2 |
| Naphthol | 82.7 |
| 4-F | 59.7 |
| 4-tBu | 59.0 |
| 4-MeO | 46.9 |

**Example 6:** (Comparative example) Preparation of 4-[3-[4-cyano-3-(trifluoromethyl)phenyl]-5,5-dimethyl-4-oxo-2-thioxo-1-imidazolidinyl]-2-fluoro-N-methyl-benzamide (Enzalutamide) following the procedure reported in example 5 of WO 2011/10657.

The reaction was performed in a jacketed glass reactor, equipped with mechanical stirrer, thermometer, condenser and kept under nitrogen atmosphere using 2 g of fragment A methyl ester (7.45 mmoles) obtainable as disclosed in WO 2011/106570.

The amount of charged compound (III a) (fragment B) was 2.0 eq with respect to Fragment A. After charging the reagents and the solvents (isopropyl acetate (IPAc) and dimethyl sulfoxide (DMSO) respectively in amount of 2 vol and 1 vol with respect to Fragment A, the mixture was heated under stirring to 83-84°C for 30 hours. The kinetic of the reaction was followed by sampling the mixtures at 10, 24 and 30 hours, and by analysing each sample by HPLC method to

determine, as reported in Table 1, the yields in solution calculated by titrating the reaction mixture with an external standard of Enzalutamide. All samples were also analysed to determine the impurities profile using HPLC, in particular to calculate the total amount of the degradation products of isothiocyanate, i.e. the sum of the area per cent of peaks related to methyl thiocarbamate, Thiourea, Urea and Trimer Guaanidine and its trend during all the experiments.

The impurities determination is reported in Table 1 as area per cent of HPLC analysis of each sample.

The titrated yields are comparable with that reported in WO 2011/10657.

| Compo und (IIIa) (equiv) | Time (hours) | Titrated Yield (%) | Unreacted fragment Amethyl ester (%) | Enzalutamide (A%) | Oxo | OpenRing (A%) | Unreacted Frag. B (A%) | Σdegradation Impurities Frag. B (A%) | Σ other Impurities (A%) |
|---|---|---|---|---|---|---|---|---|---|
| 2.0 | 10 | 87.4 | 5.3 | 63.8 | 0.2 | nd | 2.7 | 23.4 | 4.6 |
| | 24 | 91.0 | 4.3 | 66.3 | 0.2 | nd | 0.2 | 23.7 | 5.3 |
| | 30 | 91.6 | 4.1 | 66.4 | 0.3 | nd | nd | 23.5 | 5.7 |

**Example 7:** (Comparative example) Preparation of 4-[3-[4-cyano-3-(trifluoromethyl)phenyl]-5,5-dimethyl-4-oxo-2-thioxo-1-imidazolidinyl]-2-fluoro-N-methyl-benzamide (Enzalutamide) following the procedure reported in example 5 of WO 2011/10657 starting from compound (IIa).

The reactions were performed in a jacketed glass reactor, equipped with mechanical stirrer, thermometer, condenser and kept under nitrogen atmosphere using 2 g of compound II b for each trial.

The amount of compound (IIIa) (fragment B) used in each trial were 1.2 and 1.4 equivalents with respect to fragment A. After charging the reagents and the solvents (isopropyl acetate (IPAc) and dimethyl sulfoxide (DMSO) respectively in amount of 2 vol and 1 vol with respect to Fragment A"), the mixtures were heated under stirring to 83-84°C for 30 hours. The kinetic of the reactions was followed by sampling the mixtures at 10, 24 and 30 hours and by analysing each sample by HPLC method.

In the following Table 2 are collected the yield in solution calculated for each sample taken and the impurities measured from HPLC chromatogram as area per cent.

Table 2

| Compound (IIIa) (equiv) | Time (hours) | Titrated Yield (%) | Unreacted fragment A p-nitrophenyl ester (A%) | Enzalutamide (A%) | Oxo (A%) | Open Ring (A%) | Unreacted Frag. B (A%) | Σ degradation Impurities Frag. B (A%) | p-nitro phenol (A%) | Σ other Impurities (A%) |
|---|---|---|---|---|---|---|---|---|---|---|
| 1.2 | 10 | 86.2 | 8.3 | 66.9 | 0.1 | nd | 6.0 | 5.9 | 6.0 | 6.8 |
| | 24 | 90.6 | 5.7 | 72.1 | 0.1 | nd | 0.4 | 8.3 | 6.5 | 6.9 |
| | 30 | 90.7 | 5.5 | 72.4 | 0.1 | nd | nd | 8.4 | 6.5 | 7.1 |
| 1.4 | 10 | 87.2 | 3.5 | 66.9 | 0.1 | nd | 10.5 | 6.2 | 6.0 | 6..8 |
| | 24 | 92.0 | 1.2 | 73.7 | 0.1 | nd | 1.5 | 9.5 | 6.6 | 7.4 |
| | 30 | 94.2 | 1.1 | 74.1 | 0.1 | nd | 0.1 | 10.6 | 6.6 | 7.4 |

A titrated yield value comparable to that obtained in comparative example 6 was achieved using small amount of compound (IIIa) : 1.2 instead of 2.0 equivalents.

Furthermore, small amount of oxo-enzalutamide and of impurities related to the degradation of compound (IIIa) were observed if compared to the values reported on Table 1.

**HPLC analytical method for in process controls and intermediates purity**

Equipment: Agilent 1200 LC chromatograph equipped with G1314B VWD Detector.
Software: Chemstation Rev.C.01.07
Column: ACE Excel 5 super C18 250*4.6mm,5μm or equivalent
Guard column: ACE 5 C18
Column temperature: 35°C
Mobile Phase A: Transfer 0.5ml of Trifluoroacetic Acid (TFA) to 1000ml with water (0.05%TFA)
Mobile Phase B: Acetonitrile
Elution Mode: Gradient

| Time (mins.) | A (%) | B (%) |
|---|---|---|
| 0 | 75 | 25 |
| 50 | 25 | 75 |
| 50.1 | 75 | 25 |
| 55 | 75 | 25 |

Sample tray temperature: 25°C
Sample concentration _0.4 mg/mL
Injection volume: 5 μL
UV detection: 220 nm
Analysis time: 15 minutes
Total Run time: 50 minutes

Under these conditions, Retention Time (RT) of darolutamide is typically 30.5 minutes solution preparation: Values of RRt are calculated using ENZA-00 as a reference by applying the following equation:

$$RRt = \frac{Rt_i}{Rt_{Ref}}$$

Where $Rt_i$ is the retention time of a certain component and $Rt_{Ref}$ is the retention time of reference.

| **Impurity name** | **Impurity structure** | **RT HPLC (min) / RTT** | **Origin Impurity** |
|---|---|---|---|
| 4-nitro phenol (PNP) | 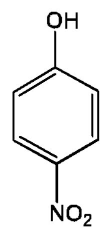 | 13.2 min /0.43 | Unreacted phenol derivative arising from fragment A" para-nitrophenyl ester when is used as reactant |

(continued)

| Impurity name | Impurity structure | RT HPLC (min) / RTT | Origin Impurity |
|---|---|---|---|
| Aniline | 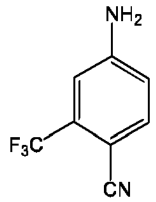 | 19.9 min / 0.65 | Hydrolysis of compound (IIIa).<br><br>It can be formed in the sample preparation from unreacted compound (IIIa). |
| Fragment A methyl ester | 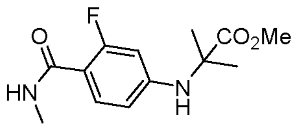 | 25.6 min/ 0.84 | Unreacted Fragment A methyl ester |
| Ring open Impurity | 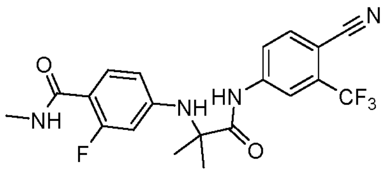 | 27.1 min/ 0.88 | Hydrolysis of an Enzalutamide synthetic intermediate |
| Oxo-Enzalutamide | 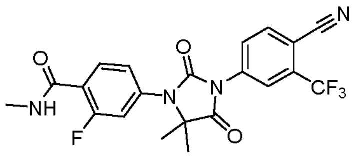 | 27.5 min/ 0.90 | Oxidation of Enzalutamide |
| Methyl Thiocarbamate | 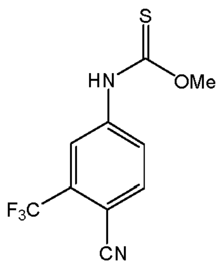 | 32.4 min/ 1.06 | From the reaction of compound (IIIa) with methanol obtained during the reaction in presence of fragment A methyl ester as reactant |
| Fragment A" para-nitrophenyl ester (compound IIIb) | 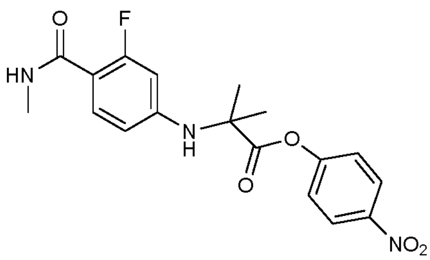 | 34.8 min/ 1.14 | Unreacted fragment A" p-nitrophenyl ester |
| Fragment B | 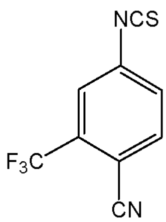 | 39.0 min /1.28 | Unreacted compound (IIIa) |

(continued)

| Impurity name | Impurity structure | RT HPLC (min) / RTT | Origin Impurity |
|---|---|---|---|
| Urea | 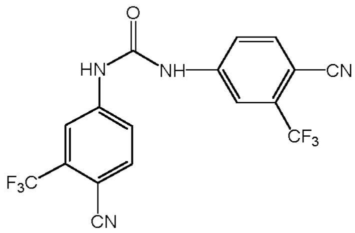 | 39.6 min/1.29 | From oxidation of Thiourea |
| Thiourea | 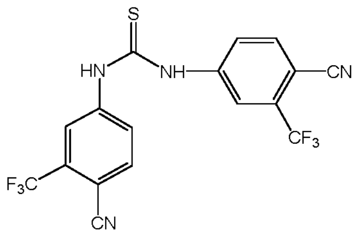 | 40.4 min/ 1.32 | From the reaction of compound (IIIa) with Aniline. It can be formed in the sample preparation from un-reacted compound (IIIa). |
| Trimer Guanidine | 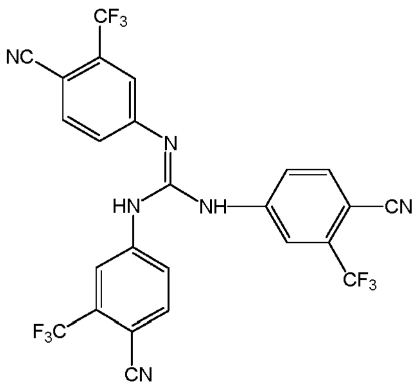 | 43.5 min/1.42 | Self-condensation of compound (IIIa). |

## Claims

**1.** A process for the preparation of a compound having the structure of formula (I)

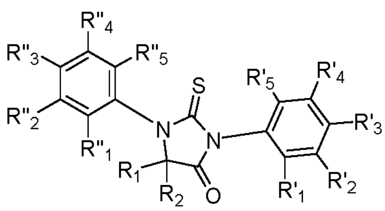

**(I)**

wherein:

- $R_1$ and $R_2$, each independently from the other, can be H, $C_1$-$C_8$-alkyl or $C_6$-$C_{10}$-aryl, optionally substituted with one or more halide, cyano, hydroxy or amino group(s);
- one or more of $R'_1$ - $R'_5$ and of $R''_1$ - $R''_5$, each independently from the others, is selected from the group consisting of $R_1$, cyano, halide, $-COOR_1$, $-CONR_1R_2$, $-OCOR_1$, $-OCNR_1R_2$, $R_1$ and $R_2$ being as defined above,

the process comprising the step of coupling the compound of formula (II) and the compound of formula (III) to obtain

the compound of formula (I)

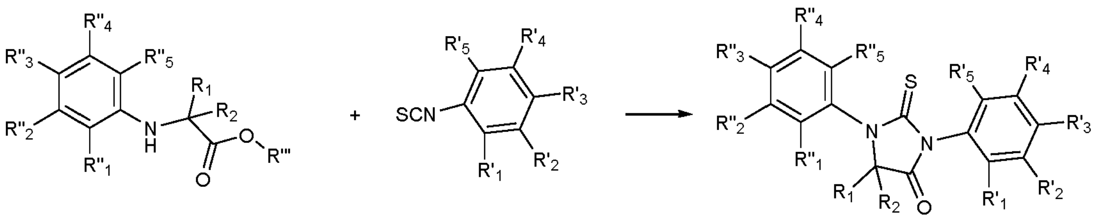

(II) (III) (I)

wherein R‴ is phenyl bearing one or more substituent(s) independently selected from halide and $-NO_2$.

2. The process according to claim 1, wherein at least one of $R_1$ and/or $R_2$ is selected from the group consisting of methyl, ethyl, propyl, isopropyl, fluorine, chlorine, bromine, iodine, trifluoromethyl and trichloromethyl.

3. The process according to any of the preceding claims, wherein at least one of $R"_1$-$R"_5$ is an halide or -CONHR1 group, wherein the halide is a fluorine group and R1 is as defined in claim 1.

4. The process according to any of the preceding claims, wherein at least one of $R'_1$-$R'_5$ is a trifluoromethyl or a cyano group.

5. The process according to any of the preceding claims, wherein compound of formula (I) is enzalutamide.

6. The process according to any of the preceding claims, wherein compound (II), have the following general formula (IIa)

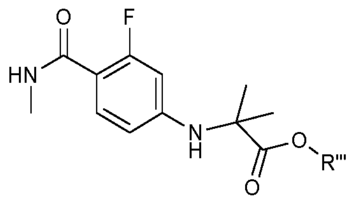

(IIa)

wherein R‴ is a phenyl bearing one or more substituent(s) independently selected from halide and $-NO_2$.

7. The process according to any of the preceding claims, wherein the step of coupling is performed with at least one of the followings: compound (II) has the following formula (IIb):

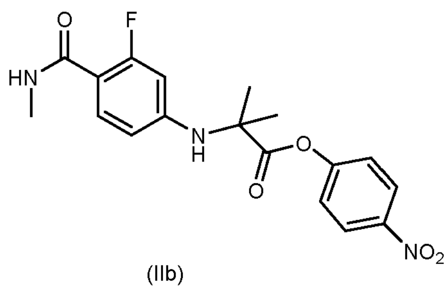

(IIb)

and compound (III) has the following formula (IIIa):

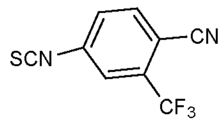

(IIIa)

**8.** The process according to claim 7, wherein in the step of coupling the compound of formula (IIb) is coupled with the compound of formula (IIIa) and 4-[3-[4-cyano-3-(trifluoromethyl)phenyl]-5,5-dimethyl-4-oxo-2-thioxo-1-imidazolidinyl]-2-fluoro-N-methyl-benzamide is obtained and, optionally, purified via crystallization.

**9.** The process according to any of the preceding claims, further comprising the step of preparing the compound of formula (II) by esterification of the corresponding carboxylic acid with a phenol derivative of formula R'''OH, wherein R''' is a phenyl bearing one or more substituent (s) independently selected from halide and $-NO_2$.

**10.** A compound of general formula (IIa):

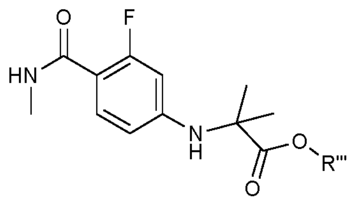

(IIa)

wherein R''' is a phenyl bearing one or more substituent(s) independently selected from halide and -NO2.

**11.** The compound according to claim 10, wherein R''' is 4-nitrophenyl, pentafluorophenyl, 4-chlorophenyl, 2,4-dichlorophenyl or 2,4-difluorophenyl, preferably wherein R''' is 4-nitrophenyl, pentafluorophenyl or 4-chlorophenyl.

**12.** The compound according to claims 10-11 having the following formula (IIb):

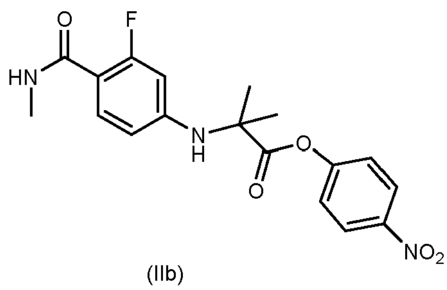

(IIb)

**13.** A process for the preparation of the compound of formula (IIa) defined in claim 10, comprising the step of esterification of the carboxylic acid of formula (IV) with a phenol derivative of formula R'''OH, wherein R''' is a phenyl bearing one or more substituent(s) independently selected from halide and $-NO_2$.

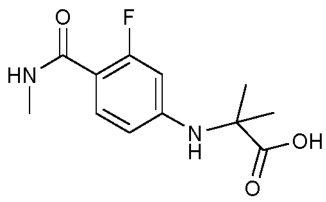

(IV)

**14.** A process for the preparation of the compound of formula (IIa) according to claim 13, wherein R‴ is selected from 4-nitrophenyl, 4-chlorophenyl and pentafluorphenyl.

**15.** Use of a compound of formula (IIa) defined in claim 10 for the preparation of enzalutamide.

**16.** Use of a compound of formula (IIb) defined in claim 12 for the preparation of enzalutamide.

## Patentansprüche

**1.** Verfahren zur Herstellung einer Verbindung mit der Struktur der Formel

(I)

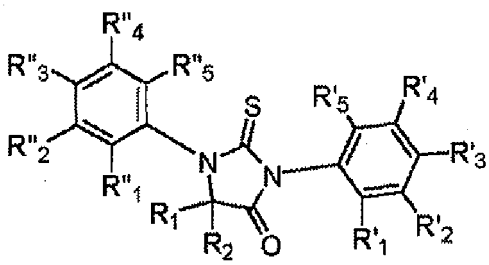

**(I)**

wobei:

- $R_1$ und $R_2$, jeweils unabhängig voneinander, H, $C_1$-$C_8$-Alkyl oder $C_6$-$C_{10}$-Aryl bedeuten können, optional substituiert mit einer oder mehreren Halogenid-, Cyano-, Hydroxy- oder Aminogruppe(n);
- einer oder mehrere von $R'_1$ - $R'_5$ und von $R''_1$ - $R''_5$, jeweils unabhängig voneinander, aus der Gruppe ausgewählt ist, bestehend aus $R_1$, Cyano, Halogenid, -$COOR_1$, -$CONR_1R_2$, -$OCOR_1$ und -$OCNR_1R_2$, wobei $R_1$ und $R_2$ wie oben definiert sind,

wobei das Verfahren den Schritt des Koppelns der Verbindung der Formel (II) und der Verbindung der Formel (III) umfasst, um die Verbindung der Formel (I) zu erhalten,

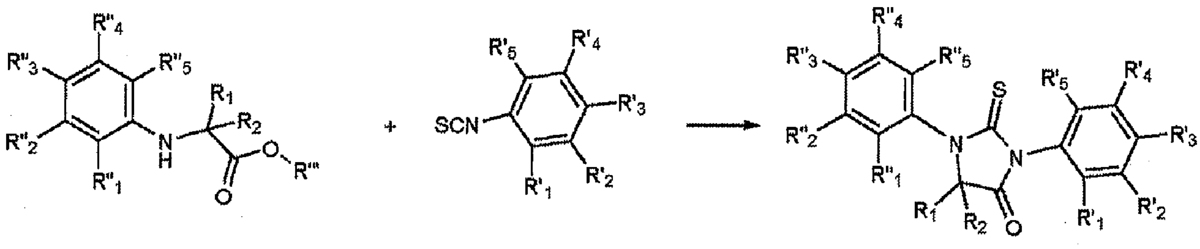

(II) (III) (I)

wobei R‴ Phenyl bedeutet, das einen oder mehrere Substituenten trägt, die unabhängig voneinander ausgewählt sind aus Halogenid und $-NO_2$.

2. Verfahren nach Anspruch 1, wobei mindestens einer von $R_1$ und/oder $R_2$ ausgewählt ist aus der Gruppe bestehend aus Methyl, Ethyl, Propyl, Isopropyl, Fluor, Chlor, Brom, Iod, Trifluormethyl und Trichlormethyl.

3. Verfahren nach einem der vorstehenden Ansprüche, wobei mindestens einer von $R''_1$ - $R''_5$ ein Halogenid oder eine $-CONHR_1$-Gruppe ist, wobei das Halogenid eine Fluorgruppe ist und $R_1$ wie in Anspruch 1 definiert ist.

4. Verfahren nach einem der vorstehenden Ansprüche, wobei mindestens einer von $R'_1$ - $R'_5$ eine Trifluormethyl- oder eine Cyanogruppe ist.

5. Verfahren nach einem der vorstehenden Ansprüche, wobei die Verbindung der Formel (I) Enzalutamid ist.

6. Verfahren nach einem der vorstehenden Ansprüche, wobei die Verbindung (II) die folgende allgemeine Formel (IIa) aufweist

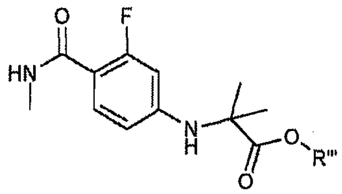

(IIa)

wobei R‴ ein Phenyl ist, das einen oder mehrere Substituenten trägt, die unabhängig voneinander ausgewählt sind aus Halogenid und $-NO_2$.

7. Verfahren nach einem der vorstehenden Ansprüche, wobei der Kupplungsschritt mit mindestens einer der Folgenden durchgeführt wird:
Verbindung (II) weist die folgende Formel (IIb) auf:

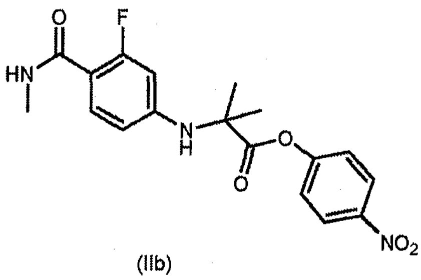

(IIb)

und Verbindung (III) weist die folgende Formel (IIIa) auf:

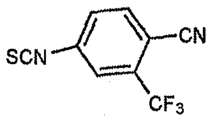

(IIIa)

8. Verfahren nach Anspruch 7, wobei im Kupplungsschritt die Verbindung der Formel (IIb) mit der Verbindung der Formel (IIIa) gekuppelt wird und 4-[3-[4-Cyano-3-(trifluormethyl)-phenyl]-5,5-dimethyl-4-oxo-2-thioxo-1-imidazolidinyl]-2-fluor-N-methylbenzamid erhalten wird und gegebenenfalls durch Kristallisation gereinigt wird.

9. Verfahren nach einem der vorstehenden Ansprüche, zusätzlich umfassend den Schritt der Herstellung der Verbindung der Formel (II) durch Veresterung der entsprechenden Carbonsäure mit einem Phenolderivat der Formel R''' OH, wobei R''' ein Phenyl ist, das einen oder mehrere Substituenten aufweist, die unabhängig voneinander aus Halogenid und $-NO_2$ ausgewählt sind.

10. Verbindung der allgemeinen Formel (IIa)

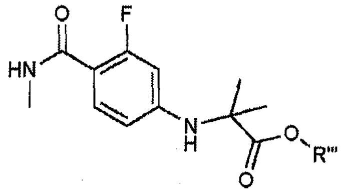

(IIa)

wobei R''' ein Phenyl ist, das einen oder mehrere Substituenten trägt, die aus Halogenid und $-NO_2$ ausgewählt sind.

11. Verbindung nach Anspruch 10, wobei R''' 4-Nitrophenyl, Pentafluorphenyl, 4-Chlorphenyl, 2,4-Dichlorphenyl oder 2,4-Difluorphenyl ist, vorzugsweise wobei R''' 4-Nitrophenyl, Pentafluorphenyl oder 4-Chlorphenyl ist.

12. Verbindung nach den Ansprüchen 10-11 mit der folgenden Formel (IIb)

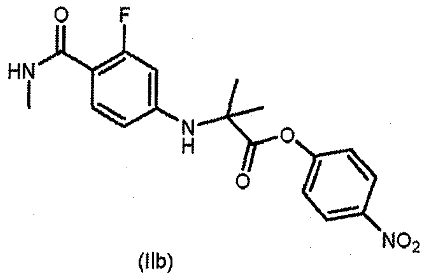

(IIb)

13. Verfahren zur Herstellung der Verbindung der Formel (IIa) nach Anspruch 10, umfassend den Schritt der Veresterung der Carbonsäure der Formel (IV)

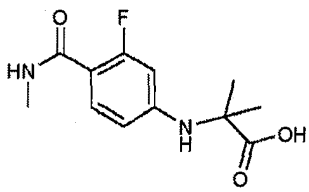

(IV)

mit einem Phenolderivat der Formel R'''OH, wobei R''' ein Phenyl ist, das einen oder mehrere Substituent(en) trägt, der/die unabhängig voneinander ausgewählt ist/sind aus Halogenid und $-NO_2$.

14. Verfahren zur Herstellung der Verbindung der Formel (IIa) nach Anspruch 13, wobei R''' ausgewählt ist aus 4-Nitrophenyl, 4-Chlorphenyl und Pentafluorphenyl.

15. Verwendung einer Verbindung der Formel (IIa), definiert wie in Anspruch 10, zur Herstellung von Enzalutamid.

16. Verwendung einer Verbindung der allgemeinen Formel (IIb), definiert wie in Anspruch 12, zur Herstellung von Enzalutamid.

## Revendications

**1.** Procédé de préparation d'un composé présentant la structure de formule (I)

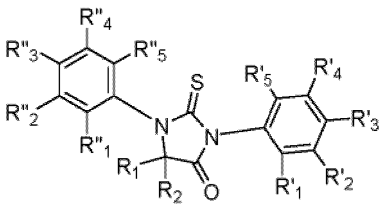

**(I)**

dans lequel :

- $R_1$ et $R_2$, indépendamment l'un de l'autre, peuvent être H, un alkyle en $C_1$-$C_8$ ou un aryle en $C_6$-$C_{10}$, facultativement substitué par un ou plusieurs groupes halogénure, cyano, hydroxy ou amino ;
- un ou plusieurs de $R'_1$ à $R'_5$ et de $R''_1$ à $R''_5$, chacun indépendamment des autres, est choisi dans le groupe constitué de $R_1$, cyano, halogénure, $-COOR_1$, $-CONR_1R_2$, $-OCOR_1$, $-OCNR_1R_2$, $R_1$ et $R_2$ étant tels que définis ci-dessus,

le procédé comprenant l'étape consistant à coupler le composé de formule (II) et le composé de formule (III) pour obtenir le composé de formule (I)

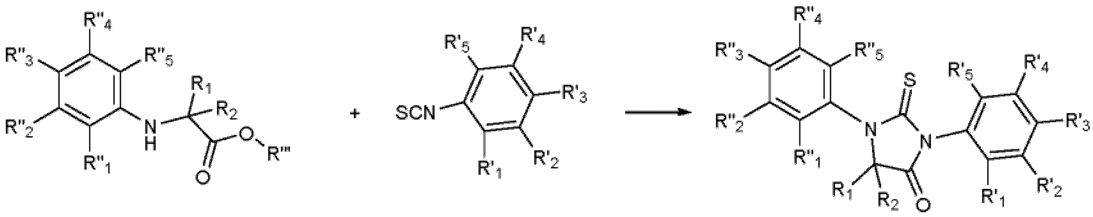

(II) (III) (I)

dans lequel R‴ est un phényle portant un ou plusieurs substituants choisis indépendamment parmi un halogénure et $-NO_2$.

**2.** Procédé selon la revendication 1, dans lequel au moins l'un de $R_1$ et/ou $R_2$ est choisi dans le groupe constitué de méthyle, éthyle, propyle, isopropyle, fluor, chlore, brome, iode, trifluorométhyle et trichlorométhyle.

**3.** Procédé selon l'une quelconque des revendications précédentes, dans lequel au moins un parmi $R''_1$ à $R''_5$ est un halogénure ou un groupe -CONHR1, dans lequel l'halogénure est un groupe fluor et $R_1$ est tel que défini dans la revendication 1.

**4.** Procédé selon l'une quelconque des revendications précédentes, dans lequel au moins un parmi $R'_1$ à $R'_5$ est un groupe trifluorométhyle ou un groupe cyano.

**5.** Procédé selon l'une quelconque des revendications précédentes, dans lequel le composé de formule (I) est l'enzalutamide.

**6.** Procédé selon l'une quelconque des revendications précédentes, dans lequel le composé (II) présente la formule générale (11a) suivante

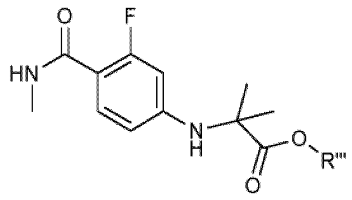

(IIa)

dans lequel R‴ est un phényle portant un ou plusieurs substituants choisis indépendamment parmi un halogénure et $-NO_2$.

**7.** Procédé selon l'une quelconque des revendications précédentes, dans lequel l'étape de couplage est réalisée avec au moins l'un des éléments suivants : le composé (II) répond à la formule (IIb) suivante :

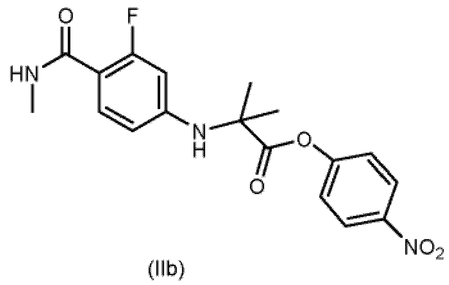

(IIb)

et le composé (III) répond à la formule (IIIa) suivante :

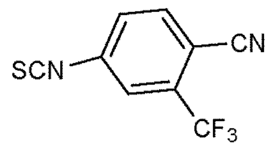

(IIIa)

**8.** Procédé selon la revendication 7, dans lequel, dans l'étape de couplage, le composé de formule (IIb) est couplé au composé de formule (IIIa) et le 4-[3-[4-cyano-3-(trifluorométhyl)phényl]-5,5-diméthyl-4-oxo-2-thioxo-1-imidazolidinyl]-2-fluoro-N-méthyl-benzamide est obtenu et, facultativement, purifié par cristallisation.

**9.** Procédé selon l'une quelconque des revendications précédentes, comprenant en outre l'étape de préparation du composé de formule (II) par estérification de l'acide carboxylique correspondant avec un dérivé de phénol de formule R‴OH, dans lequel R‴ est un phényle portant un ou plusieurs substituants choisis indépendamment parmi un halogénure et $-NO_2$.

**10.** Composé de formule générale (IIa) :

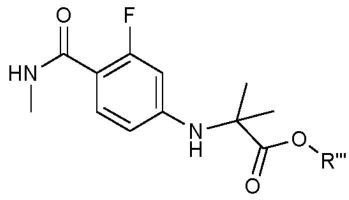

(IIa)

dans lequel R‴ est un phényle portant un ou plusieurs substituants choisis indépendamment parmi un halogénure et $-NO_2$.

**11.** Composé selon la revendication 10, dans lequel R‴ est le 4-nitrophényle, le pentafluorophényle, le 4-chlorophényle, le 2,4-dichlorophényle ou le 2,4-difluorophényle, de préférence dans lequel R‴ est le 4-nitrophényle, le pentafluorophényle ou le 4-chlorophényle.

**12.** Composé selon les revendications 10-11 répondant la formule (IIb) suivante :

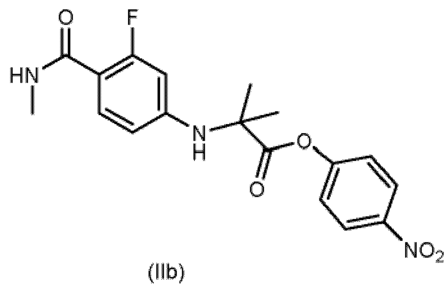

(IIb)

**13.** Procédé de préparation du composé de formule (IIa) défini dans la revendication 10, comprenant l'étape d'estérification de l'acide carboxylique de formule (IV) avec un dérivé de phénol de formule R'"OH, dans lequel R‴ est un phényle portant un ou plusieurs substituants choisis indépendamment parmi un halogénure et $-NO_2$.

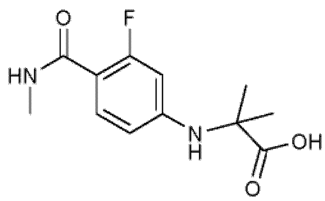

(IV)

**14.** Procédé de préparation du composé de formule (IIa) selon la revendication 13, dans lequel R‴ est choisi parmi le 4-nitrophényle, le 4-chlorophényle et le pentafluorphényle.

**15.** Utilisation d'un composé de formule (IIa) selon la revendication 10 pour la préparation d'enzalutamide.

**16.** Utilisation d'un composé de formule (IIb) selon la revendication 12 pour la préparation d'enzalutamide.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006124118 A
- WO 2011106570 A
- WO 2015121768 A
- WO 2015154730 A
- WO 201110657 A


### Non-patent literature cited in the description


- *Cryst. Growth. Des*, 2018, vol. 18 (7), 3774-3780